# EUROPEAN PATENT APPLICATION

(11) **EP 1 754 452 A1**
(43) Date of publication of application: **21.02.2007**
(21) Application number: 05701669.3
(22) Date of filing: 13.01.2005
(51) Int. Cl.: A61D 1/00

(54) **CANINE CARPAL ARTHRODESIS PLATE**

(30) Priority: 16.01.2004 ES 200400077 U
(71) Applicant: UNIVERSITAT AUTONOMA DE BARCELONA, 08193 Bellaterra (ES)
(72) Inventor: D AZ-BERTRANA S NCHEZ, Carmen, E-08193 Bellaterra (ES); DURALL RIVAS, Ignacio, E-08193 Bellaterra (ES)
(74) Representative: Barlocci, Anna
(86) International application number: PCT/ES2005/000014
(87) International publication number: WO 2005/067814

(57) **Abstract**

The invention relates to a canine carpal arthrodesis plate consisting of a long metal element comprising two arms, namely a proximal arm and a distal arm, as well as holes for the insertion of fixing screws and a central hole, the aforementioned arms being separated by an intermediate spacing at the plate application face. According to the invention, the distal arm forms a small inclination angle in relation to the proximal arm.

## Description

### Specification

The present invention is intended to provide an improved canine carpal arthrodesis plate having appreciable novelty and inventive step features over what is presently known.

As is known, the carpal arthrodesis (panarthrodesis) is the carpal-radial and carpal-cubital, intercarpal and carpal-metacarpal joint fusion. They mostly occur when serious injuries take place in the ligaments of these joints caused by traumas as a consequence of fights, falls, knocking down, etc. Other indications of the carpal panarthrodesis are joint degenerative processes (arthrosis), periarticular soft tissue injuries, painful injuries not responding to medication and resolution of the radial nerve paralysis, among others, etc.

Panarthrodeses were initially carried out with the dynamic compression plates (oval holes) with 3.5, 2.7 or 2.0 mm screws depending upon dog or cat size. Intervention consists in fixing all the carpal joints by means of a plate that is screwed to the radius, the carpic radial and the metacarpal III bone. These plates are flat and can be bent by using bending wrenches or any other plate bending tool. A hybrid dynamic compression plate for carrying out carpal panarthrodesis is published in 1999 in Veterinary Comparative Orthopaedic & Traumatology magazine. Said plate is flat although plate thickness is progressively reduced at the distal end thereof, in the two planes (dorso-ventral and latero-medial) conferring a slight cranial tilt of about 10°-15°. There is a (large sized) model that employs 3.5 screws in the entire plate. There is a medium sized model that employs 3.5 screws in the proximal arm, and in the carpic radial bone, and a 2.7 screw in the plate distal arm. Finally, the small sized model employs 2.7 mm screws in the proximal arm, and carpic radial bone and 2.0 mm screws in the distal arm. These models are made having round or oval holes except for the carpic radial bone which is always round.

The inventor has carried out tests with the presently known devices and different problems have been observed in their application. Effectively, after applying the hybrid plate in several corpses with or without coagulation, and after radiographs have been performed, it has been noticed that there is a clearance between the plate surface and the carpic radial bone (from 2.0 to 5.0 mm) depending on the animal size. When the screw is inserted into the carpic radial bone and it is tightened a cranial displacement not only of this bone but the entire terminal plantar zone takes place. It is proven that adapting the joint to the plate surface instead of adapting the plate to the join anatomy is an error.

Researches carried out by the inventor resulted in development of the improved canine carpal arthrodesis plate object of the invention which provides appreciable improvements over presently known devices.

Essentially, the plate object of the present invention is a hybrid plate having, as an essential feature, a stepped structure and a certain inclination in the axial plane as well as an enlarged hole for the screw to be inserted into the carpic radial bone. By applying the plate of the invention, it is possible to obtain much better results in treatment of the above mentioned ligament injuries, as well as joint degenerative processes, and other injuries in animals such as dogs and cats.

For a better understanding, drawings are herein attached by way of an explanatory but non limiting example showing the state of the art and a preferred embodiment of the present invention.
Figures 1, 2 and 3 show views of the application of a plate corresponding to the state of the art.
Figures 4 to 8 correspond to views in detail of the plate object of the present invention.
Figure 9 shows a view of the application of the plate object of the present invention in a correction process.
Figures 10, 11 and 12 show respective details of the plate object of the present invention.

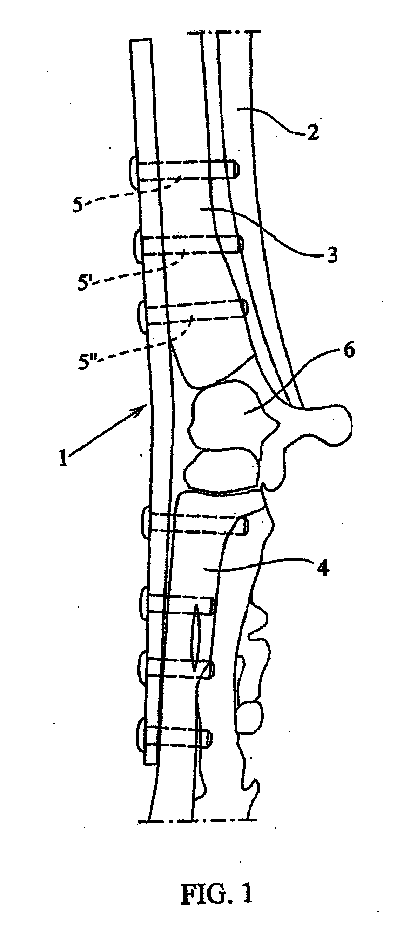

A conventional plate arrangement 1 can be seen from figure 1 corresponding to the state of the art in a dog joint, in which the ulna 2 and the radius 3 of the proximal portion, as well as the metacarpals 4 of the distal portion can be seen, the provision of a plurality of screws 5, 5', 5", .... being shown for fixing the plate to the animal's extremity. With this application, the distance from the plate to the carpic radial bone 6 is 4.0 mm.

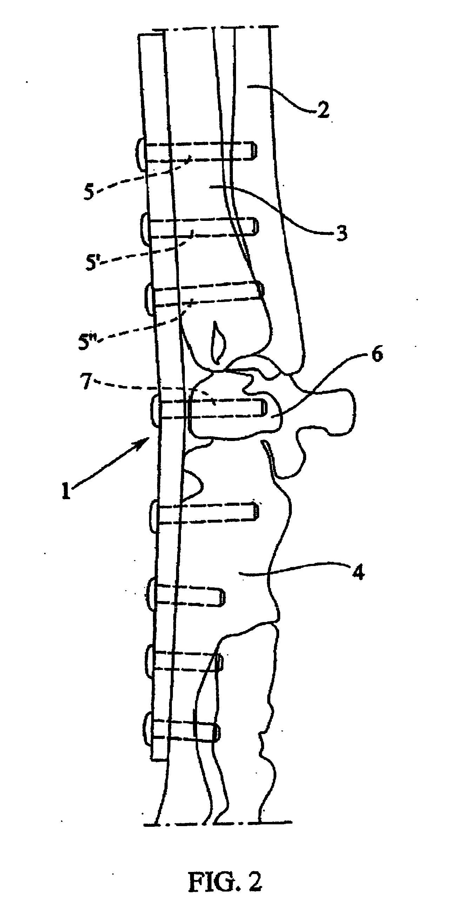

In case of applying a screw in the carpic radial bone 7, such as it can be seen in the embodiment of figure 2, in which the rest of reference numerals is equivalent to the figure 1, it can be seen that when tighten it the carpic radial bone is pulled and the contact surface between said bone and the radius articular surface are reduced and misaligned. On the other hand when tightening the metacarpal bone screws the palmar region is cranially displaced.

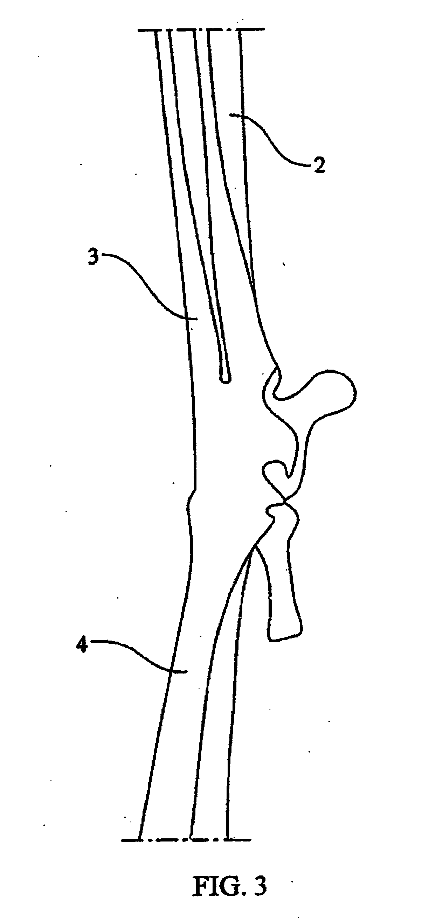

In the view in figure 3, it can be seen that the consolidation obtained is not anatomical and deambulation is not harmonious.

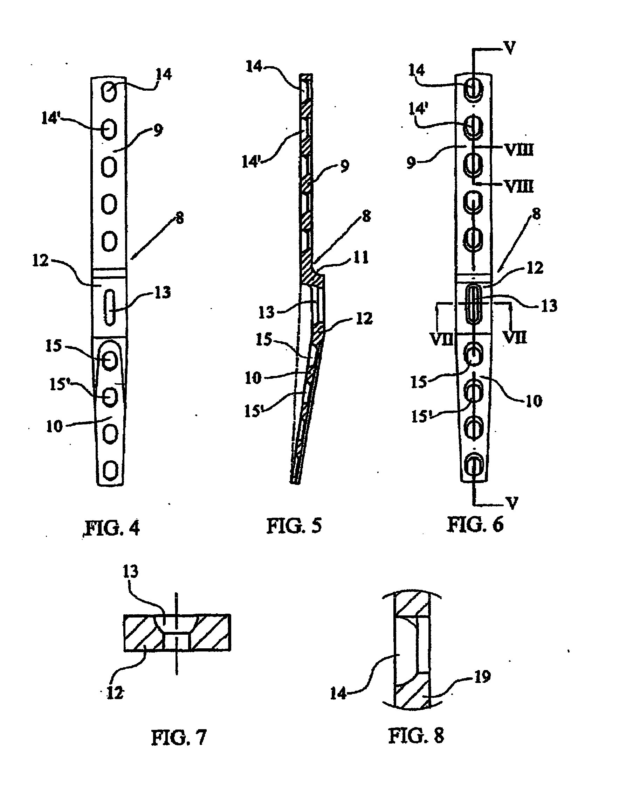

In order to overcome the above problems, the present invention provides an improved arthrodesis plate, indicated at 8 in figure 4 and subsequent figures, being characteristic that said plate has two segments 9 and 10, in the proximal and distal portion, respectively, spaced apart to each other by a step 11 in between, at least at the plate application face, giving rise to a hybrid plate which further has a certain inclination since the segment 10 is tilted relative to the central portion 12 that is straight and substantially parallel to the arm 9 of the proximal end. The face of the distal arm 10 facing the application face can be step-like shaped as shown in the figures or it can take on any other shape, being able to be straight, as shown in broken lines in figure 5.

The new arthrodesis plate has a hole with an enlarged structure for insertion of the fixing screw into the carpic radial bone, which has been indicated at 13 extending along the flat portion 12, and the end arms 9 and 10 have hole alignments 14, 14'... and 15, 15', respectively. As an alternative, the hole 13 could be round.

Even though the size and the number or holes could be variable according to design, in one preferred embodiment the arm 9 will be provided with five identical holes and the arm 10 with four holes, all of them slightly enlarged, such as it can be seen in the longitudinal cross section in figure 8. As an example of embodiment, it will be indicated that the radius of curvature of the hole 13 will be able to be 2.5 mm and 6.5 mm in length, and with a hole width of about 3 mm. Total length of the plate can be of about 143 mm and the length of the arms 70 and 73 mm, respectively, with the length of the central flat portion being of about 18 mm and the length of the central hole 9 mm, and the width thereof being of about 3 mm.

Spacing between the holes will be of about 13 mm and spacing relative to the end of about 6 mm. The angle of the inclined arm has been shown in one example to be of about 10°.

However, it has to be seen that the indicated values are merely an example and they are in no way limitative of the present invention, being able to be varied between certain limits without departing from the scope of the present invention.

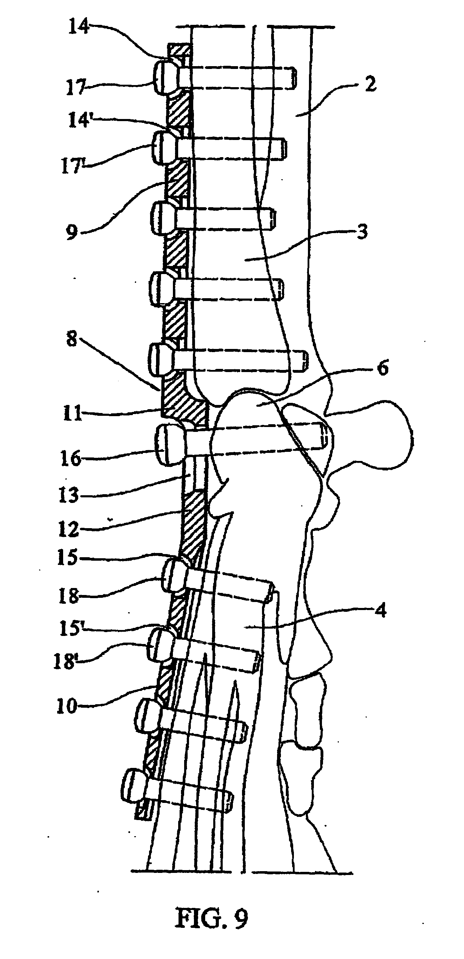

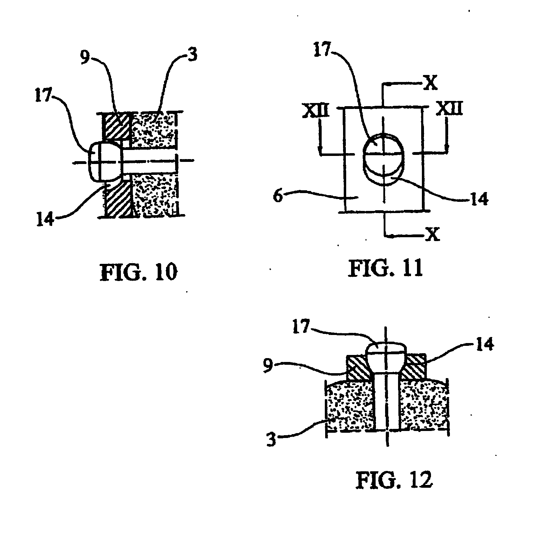

Positioning of the plate according to the present invention can be seen from figure 9 wherein a well adapted positioning of the plate in the animal's extremity where it is placed is shown, in which the rational and harmonious screw distribution can be seen, including the screw 16 of the carpic radial bone. The corresponding screws 17, 17',..., and 18, 18'..., have, in the segments 9 and 10, a spherical head for being received into the holes having the above mentioned structure and which can be seen in greater detail in figures 10 to 12.

Within the essence of the invention, there is the possibility of a likewise protected detail embodiment and thereby the shape of the proximal and/or distal portion outline, the amount of round or oval holes and of course size and materials in which it is made could be any.

Everything that do not affect, alter, change or modify the essence of the disclosed plate will be variable for the purpose of the present invention.

## Claims

1. Canine carpal arthrodesis plate, of the type comprising a long metal element having two end arms, proximal and distal, with holes for the insertion of fixing screws and a central hole, **characterized in that** said end arms are separated by an intermediate stepping at least at the plate application face.

2. Canine carpal arthrodesis plate as claimed in claim 1, wherein the distal arm forms a small inclination angle in relation to the proximal arm.

3. Canine carpal arthrodesis plate as claimed in claim 1, wherein the arm having inclination starts from a short flat area of joining with the intermediate stepping.

4. Canine carpal arthrodesis plate as claimed in claim 1, wherein the central hole of fixation to carpal bone is enlarged with concave walls having a spherical cross section intended to receive the spherical headed fixing screws.
